# EUROPÄISCHE PATENTSCHRIFT

(11) **EP 0 923 526 B1**
(45) Veröffentlichungstag und Bekanntmachung des Hinweises auf die Patenterteilung: **03.04.2002**
(21) Anmeldenummer: 97937547.4
(22) Anmeldetag: 01.08.1997
(51) Int. Cl.: C07C 57/04, C07C 51/48

(54) **VERFAHREN ZUR ABTRENNUNG VON ACRYLSÄURE**
PROCESS FOR THE SEPARATION OF ACRYLIC ACID
PROCEDE DE SEPARATION D'ACIDE ACRYLIQUE

(30) Priorität: 05.08.1996 DE 19631645
(43) Veröffentlichungstag der Anmeldung: 23.06.1999
(73) Patentinhaber: BASF AKTIENGESELLSCHAFT, 67056 Ludwigshafen (DE)
(72) Erfinder: HEIDA, Bernd, D-67158 Ellerstadt (DE); THIESSEN, Fritz, D-67071 Ludwigshafen (DE); HAMMON, Ulrich, D-68165 Mannheim (DE); DAMS, Albrecht, D-67157 Wachenheim (DE)
(74) Vertreter: Isenbruck, Günter, Dr.
(86) Internationale Anmeldenummer: EP9704214
(87) Internationale Veröffentlichungsnummer: WO9805622

(56) Entgegenhaltungen:
- DE-A- 2 136 396
- DE-A- 2 449 780
- DE-A- 4 308 087

## Beschreibung

Die vorliegende Erfindung bezieht sich auf ein Verfahren zur Gewinnung von Acrylsäure bei der katalytischen Gasphasenoxidation, insbesondere auf eine Rückgewinnung von Acrylsäure aus dem anfallenden Sauerwasser.

Acrylsäure kann durch katalytische Gasphasenoxidation von Propen hergestellt werden. Dabei kann das zu oxidierende Propen mit einem Verdünnungsgas oder mit einem nicht umgesetzte Edukte enthaltenden Kreisgas vermischt sein. Das Reaktionsgemisch der Gasphasenoxidation wird zur Abtrennung der Acrylsäure in eine Absorptionskolonne geleitet. Dort wird die Acrylsäure mit Hilfe von Lösungsmitteln wie etwa Lactamen, zum Beispiel Methylpyrrolidon, oder anderen organischen Säuren, zum Beispiel Ethylhexansäure, weitgehend aus dem Gemisch von Reaktionsprodukten abgetrennt. Die nicht absorbierten Komponenten werden gasförmig aus der Absorptionskolonne entnommen und einer Kondensationsstufe zugeführt. Der kondensierbare und kondensierte Teil dieses Gasgemischs wird abgetrennt und als sogenanntes Sauerwasser abgeleitet. Der nicht kondensierbare gasförmige Teil wird dagegen teilweise wieder in die Gasphasenoxidation zurückgeführt und bildet das sogenannte Kreisgas. Das Sauerwasser wird im Regelfall verbrannt. Damit werden zwar viele Leicht- und Mittelsieder aus dem Produktionskreislauf entfernt, es wird aber auch in dem Sauerwasser enthaltene Acrylsäure vernichtet.

Dieser Verlust an Acrylsäure kann dadurch vermieden werden, daß das Sauerwasser destilliert oder mit geeigneten Verbindungen extrahiert wird. Als geeignete Verbindungen zur Extraktion von Acrylsäure aus wäßrigen Lösungen geben I.M. Korenman et al., "Distribution of acrylic acid between organic solvents and water" (Übersetzung von Consultants Bureau, New York, aus Zhurnal Prikladnoi Khimii, Bd. 45, Nr. 5, S. 1078-1082, Mai 1972), unter anderem Dimethylphthalat an. Die Destillation hat den Nachteil eines hohen Energieverbrauchs, die Extraktion führt zu einem nicht unerheblichen zusätzlichen technischen Aufwand, da neben der Extraktion des Sauerwassers auch eine Destillation oder Strippung des Lösungsmittels aus der Extraktphase der Sauerwasserextraktion vorgesehen werden muß. Dieser Kreislauf zur Rückgewinnung der Acrylsäure aus dem Sauerwasser wird vor allem dadurch aufwendig, daß eine Verschleppung des dort verwendeten Lösungsmittels in den Herstellungskreislauf möglichst vermieden werden muß. Die Anwesenheit weiterer organischer Lösungsmittel im Herstellungskreislauf kann nämlich sowohl die quantitative Ausbeute, wie auch die Qualität der erhaltenen Acrylsäure negativ beeinflussen. Es ist sehr aufwendig, derartige negative Effekte sicher auszuschließen.

Aus DE-A-43 08 087 und DE-A-21 36 396 sind Verfahren zur Gewinnung/ Abtrennung von Acrylsäure aus den Reaktionsgasen der katalytischen Oxidation von Propen bekannt. Dabei kann in einer ersten Absorptionsstufe im Gegenstrom eine Mischung aus Diphenylether, Diphenyl und o-Dimethylphthalat eingesetzt werden.

Aufgabe der vorliegenden Erfindung ist es deshalb, ein effizientes Verfahren zur Herstellung von Acrylsäure zu finden, bei dem zusätzlich Acrylsäure aus dem Sauerwasser auf einfache und wirksame Weise zurückgewonnen werden kann.

Die Lösung geht aus von dem Verfahren zur Gewinnung von Acrylsäure bei der katalytischen Gasphasenoxidation von Propen, wobei aus dem Reaktionsgemisch der Gasphasenoxidation Acrylsäure in einer Absorptionsstufe mit Hilfe eines ersten Lösungsmittels, das mindestens ein substituiertes oder unsubstituiertes Diphenyl, einen substituierten oder unsubstituierten Diphenylether oder Dimethylphthalat enthält, absorbiert wird, aus der Absorptionsstufe ein Gasgemisch entnommen wird, das an dem ersten Lösungsmittel und an Acrylsäure arm ist, in einer Kondensationsstufe das Gasgemisch, vorzugsweise auf eine Temperatur von 20 °C bis 60 °C, abgekühlt wird, die kondensierte Phase des Gasgemisches aus der Kondensationsstufe als Sauerwasser entnommen wird und die gasförmige Phase des Gasgemisches aus der Kondensationsstufe entnommen und zumindest teilweise als Kreisgas zur Gasphasenoxidation zurückgeführt wird. Das erfindungsgemäße Verfahren ist dadurch gekennzeichnet, daß in einer Sauerwasser-Extraktionsstufe Acrylsäure aus dem Sauerwasser mit Hilfe eines zweiten Lösungsmittels, das mindestens ein substituiertes oder unsubstituiertes Diphenyl, einen substituierten oder unsubstituierten Diphenylether oder Dimethylphthalat enthält, extrahiert wird.

Vorzugsweise wird als erstes Lösungsmittel eine Mischung verwendet, die Diphenyl und Diphenylether und/oder Dimethylphthalat enthält, insbesondere eine, die 60 bis 100, vorzugsweise 70 bis 90 Gew.-% substituiertes oder unsubstituiertes Diphenyl und substituierten oder unsubstituierten Diphenylether und 0 bis 40, vorzugsweise 10 bis 30 Gew-% Dimethylphthalat enthält. Besonders bevorzugt sind sowohl das Diphenyl, wie auch der Diphenylether unsubstituiert. Dabei beträgt das Gewichtsverhältnis von Diphenylether zu Diphenyl vorzugsweise von 4:1 bis 2:1. Ein besonders bevorzugtes Lösungsmittel ist ein Gemisch, das von 40 bis 80 Gew.-% unsubstituiertes Diphenylether, von 10 bis 30 Gew.-% unsubstituierten Diphenyl und von 10 bis 30 Gew.-% Dimethylphthalat enthält. Darüber hinaus kann das Lösungsmittel noch weitere Bestandteile enthalten, zum Beispiel zur Steigerung der Selektivität des verwendeten Extraktionsmittels.

Aus der Absorptionsstufe wird ein Gasgemisch entnommen, das arm an dem ersten Lösungsmittel und an Acrylsäure ist. Die Konzentration von Acrylsäure in dem Gasgemisch beträgt typischerweise unter 1 Gew.-%, vorzugsweise unter 0,5 Gew.-%. Dieses Gasgemisch wird in einer Kondensationsstufe, vorzugsweise auf 20 °C bis 60 °C, abgekühlt. Die kondensierte Phase des Gasgemischs wird aus der Kondensationsstufe als Sauerwasser entnommen, die gasförmige Phase des Gasgemischs wird aus der Kondensationsstufe entnommen und zumindest teilweise als Kreisgas zur Gasphasenoxidation zurückgeführt. Gemäß der vorliegenden Erfindung wird anschließend in einer Sauerwasser-Extraktionsstufe Acrylsäure aus dem Sauerwasser mit Hilfe eines zweiten Lösungsmittels, das mindestens ein substituiertes oder unsubstituiertes Diphenyl, einen substituierten oder unsubstituierten Diphenylether oder Dimethylphthalat enthält, extrahiert. Das zweite Lösungsmittel enthält vorzugsweise mindestens 40, besonders bevorzugt mindestens 70 Gew.-% Dimethylphthalat. Das erfindungsgemäße Verfahren bietet den Vorteil, daß bei effizienter Extraktion der Acrylsäure aus dem Sauerwasser nicht darauf geachtet werden muß, ob das zweite Lösungsmittel in den Herstellungskreislauf verschleppt wird. Man verwendet nämlich sowohl im ersten, wie auch im zweiten Lösungsmittel Komponenten, die die Produktqualität bzw. die Ausbeute nicht negativ beeinflussen. Eine begrenzte Verschleppung kann sogar erwünscht sein und dazu benutzt werden, die Zusammensetzung des ersten Lösungsmittels, die sich während des Verfahrens ändert, gezielt zu beeinflussen.

Vorzugsweise wird das Sauerwasser vor der Sauerwasser-Extraktionsstufe einer Vorextraktionsstufe zugeführt, in der mit Hilfe von Sauerwasser als Extraktionsmittel Mittelsieder, zum Beispiel Maleinsäureanhydrid, aus dem ersten Lösungsmittel extrahiert werden. Regelmäßig wird nur ein Teilstrom des ersten Lösungsmittels dieser Vorextraktion von Mittelsiedern zugeführt. Die Acrylsäurekonzentration im Sauerwasser kann durch diese Extraktion leicht sinken. Das Extrakt, d.h. die wäßrige Phase, der Vorextraktionsstufe wird dann erfindungsgemäß der Sauerwasser-Extraktionsstufe zugeführt.

Weiter bevorzugt ist eine Variante des erfindungsgemäßen Verfahrens, bei dem Acrylsäure aus der Extraktphase der Sauerwasser-Extraktionsstufe mit Kreisgas oder mit Teilmengen, das heißt mit Teilströmen von gleicher oder unterschiedlicher Zusammensetzung wie das gesamte Kreisgas, oder mit Bestandteilen des Kreisgases, zum Beispiel Stickstoff oder Kohlenstoffoxiden, desorbiert wird. Es kann aber auch Stickstoff oder Luft als Desorptionsmittel verwendet werden. Die bevorzugte Verwendung von Kreisgas hat den Vorteil, daß kein zusätzliches Desorptionsmittel bereitgestellt und auf Verträglichkeit mit den anderen im Kreislauf befindlichen Komponenten getestet werden muß. Gegenüber einer Destillation weist eine Desorption außerdem grundsätzlich den Vorteil auf, daß weder ein Verdampfer, noch ein Kondensator vorgesehen werden müssen, was den Bauaufwand einer Acrylsäureproduktionsanlage erheblich vermindert.

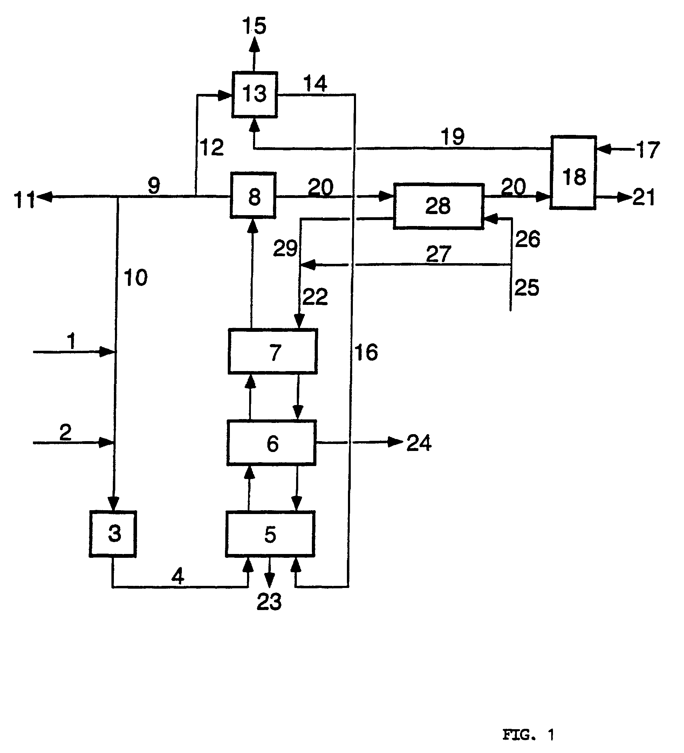

Die vorliegende Erfindung wird im folgenden anhand der Figur 1, einer Prinzipskizze eines erfindungsgemäßen Verfahrens zur Herstellung von Acrylsäure, näher beschrieben.

Propen über Leitung 1, ein Verdünnungsgas (Kreisgas oder Wasserdampf), zum Beispiel Luft oder Wasserdampf über Leitung 10 und Luft über Leitung 2 werden einem Reaktor 3 zugeführt, in dem die katalytische Gasphasenoxidation von Propen abläuft. Das dabei entstehende Acrolein kann in einem weiteren, nicht dargestellten Reaktor oxidiert werden. Über Leitung 4 gelangt das Reaktionsgemisch der Gasphasenoxidation in Quenchapparat 5. Dort wird das Reaktionsgemisch abgekühlt und ein Teil des Absorptionsmittels (Lösungsmittel) verdampft, das über Leitung 22 der Absorptionskolonne 7 zugeführt wird und von dort über Kühleinheit 6 zum Quenchapparat 5 geleitet wird. Schwersiedende Nebenkomponenten des Lösungsmittels werden im Quenchapparat 5 kondensiert und über Entnahmeleitung 23 abgezogen. Sie werden, gegebenenfalls nach einer Abdestillation von Lösungsmittel, entsorgt, zum Beispiel verbrannt. Das bereits stark abgekühlte Reaktionsgemisch wird vom Quenchapparat 5 weiter zur Kühleinheit 6, die zum Beispiel aus Kühlkreisläufen besteht, geleitet, wo es auf die geeignete Absorptionstemperatur gekühlt wird. Anschließend wird das Reaktionsgemisch in die Absorptionskolonne 7 geleitet. Dort wird durch Gegenstromabsorption mit dem über Leitung 22 zugeführten Lösungsmittel Acrylsäure aus dem Reaktionsprodukt der Gasphasenoxidation abgetrennt. Dieses Lösungsmittel kann zum Beispiel aus einem Gemisch aus etwa 60 Gew.-% unsubstituiertem Diphenylether, 20 Gew.-% unsubstituiertem Diphenyl und 20 Gew.-% Dimethylphthalat bestehen. Es kann darüber hinaus auch weitere polare Lösungsmittel zur Reduktion des Feststoffanfalls oder auch Komponenten zur Erhöhung der Selektivität aufweisen. Das mit Acrylsäure beladene Lösungsmittel wird in Kühleinheit 6 geleitet und aus dieser über Seitenabzug 24 zur weiteren, hier nicht dargestellten Aufbereitung abgezogen. Diese Aufbereitung enthält regelmäßig eine Leichtsiederstrippung und notwendigerweise eine Lösungsmitteldestillation oder einen gleichwertigen Schritt.

Die von Acrylsäure weitgehend befreiten Reaktionsprodukte werden aus der Absorptionskolonne 7 über Kopf abgezogen und in den Quenchapparat 8 geleitet. Dort wird der nicht-kondensierbare Teil über Leitung 9 entnommen und, nach der Abtrennung und Ableitung von Inertgaskomponenten über Leitung 11, als Kreisgas über Leitung 10 zur Gasphasenoxidation von Propen zurückgeführt. Dieses Kreisgas enthält unter anderem nicht umgesetzte Edukte der Gasphasenoxidation, Stickstoff und Kohlenstoffoxide. Der kondensierbare Teil der von Acrylsäure befreiten Reaktionsprodukte wird über Leitung 20 entnommen. Dieses als Sauerwasser bezeichnete Kondensat besteht aus einer wäßrigen Lösung, die neben Acrylsäure noch relevante Mengen an Essigsäure, Maleinsäure und Formaldehyd, sowie weitere Säuren enthält.

Gemäß vorliegender Erfindung wird das Sauerwasser zur Rückgewinnung eines erheblichen Anteils der darin noch enthaltenen Acrylsäure über Leitung 20 einer Extraktionsstufe 18 zugeleitet. Dort nimmt das über Leitung 17 zugeführte zweite Lösungsmittel die Acrylsäure auf. Dieses Extraktionsmittel kann zum Beispiel aus Dimethylphthalat bestehen oder substantielle Mengen davon enthalten. Das mit Acrylsäure beladene Extraktionsmittel wird über Leitung 19 zur Stripperkolonne 13 geleitet, wo über Leitung 12 zugeführtes Kreisgas die Acrylsäure vom Extraktionsmittel trennt. Das mit Acrylsäure beladene Extrakt kann dann über Leitung 14 in Quenchapparat 5 zurückgeführt werden. Die Rückführung kann aber auch an jeder anderen geeigneten Stelle im Verfahren erfolgen. Das Extraktionsmittel wird aus der Stripperkolonne 13 über Leitung 15 abgeführt und kann als Lösungsmittel wiederverwendet werden, indem es in Leitung 17 zurückgespeist wird.

Vor der Sauerwasser-Extraktion in Extraktionsstufe 18 wird das Sauerwasser aus Quenchapparat 8 vorzugsweise, wie dargestellt, einer in Leitung 20 angeordneten Sauerwasser-Vorextraktionsstufe 28 zugeführt. Dort werden mit Hilfe des Sauerwassers aus einem Teilstrom 26 des über Leitung 25 herangeführten ersten Lösungsmittels, das von der Lösungsmitteldestillation oder anderen Aufbereitungsschritten zurückgeführt wird, Mittelsieder, insbesondere Maleinsäure aus dem ersten Lösungsmittel extrahiert. Der Teilstrom 26 umfaßt typischerweise etwa 10% des Volumens des Hauptstroms 25 des Lösungsmittels. Der mit Mittelsiedern abgereicherte Teilstrom 29 des ersten Lösungsmittels wird dann, zusammen mit dessen Hauptstrom 27, über Leitung 22 der Absorptionskolonne 7 zugeleitet. Das Sauerwasser wird über den zweiten Teil von Leitung 20 der Extraktionsstufe 18 zugeleitet.

Das erfindungsgemäße Verfahren bietet somit bei minimalem technischem Aufwand und ohne Verwendung verfahrensfremder Substanzen eine höhere Ausbeute an Acrylsäure, weil die Acrylsäureverluste über das Sauerwasser nahezu vollständig beseitigt werden können.

## Patentansprüche

1. Verfahren zur Gewinnung von Acrylsäure bei der katalytischen Gasphasenoxidation von Propen, wobei
aus dem Reaktionsgemisch der Gasphasenoxidation Acrylsäure in einer Absorptionsstufe mit Hilfe eines ersten Lösungsmittels, das mindestens ein substituiertes oder unsubstituiertes Diphenyl, einen substituierten oder unsubstituierten Diphenylether oder Dimethylphthalat enthält absorbiert wird, aus der Absorptionsstufe ein Gasgemisch entnommen wird, das an dem ersten Lösungsmittel und an Acrylsäure arm ist,
in einer Kondensationsstufe das Gasgemisch, vorzugsweise auf eine Temperatur von 20 °C bis 60 °C, abgekühlt wird,
die kondensierte Phase des Gasgemisches aus der Kondensationsstufe als Sauerwasser entnommen wird und
die gasförmige Phase des Gasgemisches aus der Kondensationsstufe entnommen und zumindest teilweise als Kreisgas zur Gasphasenoxidation zurückgeführt wird,
**dadurch gekennzeichnet, daß** in einer Sauerwasser-Extraktionsstufe Acrylsäure aus dem Sauerwasser mit Hilfe eines zweiten Lösungsmittels, das mindestens ein substituiertes oder unsubstituiertes Diphenyl, einen substituierten oder unsubstituierten Diphenylether oder Dimethylphthalat enthält, extrahiert wird.

2. Verfahren nach Anspruch 1, **dadurch gekennzeichnet, daß** das erste Lösungsmittel substituiertes oder unsubstituiertes Diphenyl und substituierten oder unsubstituierten Diphenylether und/oder Dimethylphthalat enthält.

3. Verfahren nach Anspruch 1 oder 2, **dadurch gekennzeichnet, daß** Acrylsäure aus der Extraktphase der Sauerwasser-Extraktionsstufe mit Luft oder Stickstoff, oder vorzugsweise Kreisgas desorbiert wird.

4. Verfahren nach einem der Ansprüche 1 bis 3, **dadurch gekennzeichnet, daß** als erstes Lösungsmittel ein Gemisch verwendet wird, das
i) 60 bis 100, vorzugsweise 70 bis 90 Gew.-% substituiertes oder unsubstituiertes Diphenyl und substituierten oder unsubstituierten Diphenylether, und
ii) 0 bis 40, vorzugsweise 10 bis 30 Gew-% Dimethylphthalat enthält.

5. Verfahren nach Anspruch 4, **dadurch gekennzeichnet, daß** als erstes Lösungsmittel ein Gemisch verwendet wird, das
i) 40 bis 80 Gew.-% unsubstituierten Diphenylether,
ii) 10 bis 30 Gew.-% unsubstituiertes Diphenyl, und
iii) 10 bis 30 Gew.-% Dimethylphthalat
enthält.

6. Verfahren nach einem der Ansprüche 1 bis 5, **dadurch gekennzeichnet, daß** das zweite Lösungsmittel mindestens 40, vorzugsweise mindestens 70 Gew.-% Dimethylphthalat enthält.

7. Verfahren nach einem der Ansprüche 1 bis 6, **dadurch gekennzeichnet, daß** das Sauerwasser vor der Sauerwasser-Extraktionsstufe einer Vorextraktionsstufe zugeführt wird, in der mit Hilfe des Sauerwassers Mittelsieder, insbesondere Maleinsäureanhydrid, aus dem ersten Lösungsmittel extrahiert werden, und das Extrakt der Vorextraktionsstufe der Sauerwasser-Extraktionsstufe zugeführt wird.

## Claims

1. A process for obtaining acrylic acid during the catalytic gas phase oxidation of propene,
acrylic acid being absorbed from the gas phase oxidation reaction mixture in an absorption stage using a first solvent which comprises at least one substituted or unsubstituted biphenyl, a substituted or unsubstituted diphenyl ether or dimethyl phthalate, a gas mixture being taken off from the absorption stage, which mixture is low in the first solvent and in acrylic acid,
the gas mixture being cooled in a condensation stage, preferably to a temperature of from 20°C to 60°C,
the condensed phase of the gas mixture from the condensation stage being taken off as acid water and
the gaseous phase of the gas mixture being taken off from the condensation stage and at least in part recycled as circulated gas to the gas phase oxidation,
wherein acrylic acid is extracted from the acid water in an acid water extraction stage using a second solvent which comprises at least one substituted or unsubstituted biphenyl, a substituted or unsubstituted diphenyl ether or dimethyl phthalate.

2. A process as claimed in claim 1, wherein the first solvent comprises substituted or unsubstituted biphenyl and substituted or unsubstituted diphenyl ether and/or dimethyl phthalate.

3. A process as claimed in claim 1 or 2, wherein acrylic acid is desorbed from the extract phase of the acid water extraction stage using air or nitrogen, or preferably circulated gas.

4. A process as claimed in one of claims 1 to 3, wherein, as first solvent, use is made of a mixture which comprises
i) from 60 to 100, preferably from 70 to 90, % by weight of substituted or unsubstituted biphenyl and substituted or unsubstituted diphenyl ether, and
ii) from 0 to 40, preferably from 10 to 30, % by weight of dimethyl phthalate.

5. A process as claimed in claim 4, wherein, as first solvent, use is made of a mixture which comprises
i) from 40 to 80% by weight of unsubstituted diphenyl ether,
ii) from 10 to 30% by weight of unsubstituted biphenyl, and
iii) from 10 to 30% by weight of dimethyl phthalate.

6. A process as claimed in one of claims 1 to 5, wherein the second solvent comprises at least 40, preferably at least 70, % by weight of dimethyl phthalate.

7. A process as claimed in one of claims 1 to 6, wherein the acid water, upstream of the acid water extraction stage, is fed to a preextraction stage in which medium-boilers, in particular maleic anhydride, are extracted from the first solvent using the acid water, and the extract of the preextraction stage is fed to the acid water extraction stage.

## Revendications

1. Procédé de production d'acide acrylique lors de l'oxydation catalytique en phase gazeuse de propène, dans lequel de l'acide acrylique est absorbé en une étape d'absorption à partir du mélange réactionnel de l'oxydation en phase gazeuse à l'aide d'un premier solvant, qui contient au moins un diphényle substitué ou non substitué, un éther diphénylique substitué ou non substitué ou du phtalate de diméthyle, un mélange gazeux, qui est pauvre en le premier solvant et en acide acrylique, est prélevé de l'étape d'absorption, le mélange gazeux est refroidi dans une étape de condensation, de préférence à une température de 20°C à 60°C, la phase condensée du mélange gazeux est prélevée de l'étape de condensation sous la forme d'eau acide, et la phase gazeuse du mélange gazeux de l'étape de condensation est prélevée et recyclée au moins partiellement sous la forme d'un gaz de circulation vers l'oxydation en phase gazeuse,
**caractérisé en ce que**, dans une étape d'extraction d'eau acide, de l'acide acrylique est extrait de l'eau acide à l'aide d'un deuxième solvant, qui contient au moins un diphényle substitué ou non substitué, un éther diphénylique substitué ou non substitué ou du phtalate de diméthyle.

2. Procédé suivant la revendication 1, **caractérisé en ce que** le premier solvant contient du diphényle substitué ou non substitué et de l'éther diphénylique substitué ou non substitué et/ou, du phtalate de diméthyle.

3. Procédé suivant l'une des revendications 1 et 2, **caractérisé en ce que** l'acide acrylique de la phase extraite de l'étape d'extraction d'eau acide est désorbé avec de l'air ou de l'azote, ou de préférence du gaz de circulation.

4. Procédé suivant l'une des revendications 1 à 3, **caractérisé en ce que**, comme premier solvant, on utilise un mélange qui contient
i) 60 à 100, de préférence 70 à 90, % en poids de diphényle substitué ou non substitué et d'éther diphénylique substitué ou non substitué, et
ii) 0 à 40, de préférence 10 à 30, % en poids de phtalate de diméthyle.

5. Procédé suivant la revendication 4, **caractérisé en ce qu'**on utilise, comme premier solvant, un mélange qui contient
i) 40 à 80% en poids d'éther diphénylique non substitué,
ii) 10 à 30% en poids de diphényle non substitué, et
iii) 10 à 30% en poids de phtalate de diméthyle.

6. Procédé suivant l'une des revendications 1 à 5, **caractérisé en ce que** le deuxième solvant contient au moins 40, de préférence au moins 70, % en poids de phtalate de diméthyle.

7. Procédé suivant l'une des revendications 1 à 6, **caractérisé en ce que** l'eau acide est amenée avant l'étape d'extraction d'eau acide à une étape de préextraction, dans laquelle des substances à point d'ébullition moyen, en particulier de l'anhydride maléique, sont extraites à partir du premier solvant à l'aide de l'eau acide, et l'extrait de l'étape de préextraction est amené à l'étape d'extraction d'eau acide.
